# EUROPEAN PATENT SPECIFICATION

(11) **EP 3 528 900 B1**
(45) Date of publication and mention of the grant of the patent: **13.05.2020**
(21) Application number: 17783482.7
(22) Date of filing: 11.10.2017
(51) Int. Cl.: A61Q 5/06, A61K 8/04, B05B 7/04, B05B 1/34, B65D 83/62, B65D 83/14

(54) **COMPRESSED HAIR SPRAY**
KOMPRIMIERTES HAARSPRAY
LAQUE SOUS PRESSION POUR CHEVEUX

(30) Priority: 19.10.2016 EP 16194542
(43) Date of publication of application: 28.08.2019
(73) Proprietor: Unilever PLC, London, Greater London EC4Y 0DY (GB); Unilever N.V., 3013 AL Rotterdam (NL)
(72) Inventor: BROWN, Gillian, Bebington Wirral Merseyside CH63 3JW (GB); FRANKLIN, Laura, Bebington Wirral Merseyside CH63 3JW (GB); GRIFFITHS, Llyr, Glyndwr, Bebington Wirral Merseyside CH63 3JW (GB); JOINSON, Leslie, Joseph, Luke, Bebington Wirral Merseyside CH63 3JW (GB); SMITH, Jonathan, Douglas, William, Bebington Wirral Merseyside CH63 3JW (GB); SMITH, Paul, James, Bebington Wirral Merseyside CH63 3JW (GB)
(74) Representative: Mathai, Neenu Grace
(86) International application number: PCT/EP2017/075933
(87) International publication number: WO 2018/073070

(56) References cited:
- US-A- 4 940 171
- US-A1- 2014 348 770
- Anonymous: "ACTUATORS FOR AEROSOL VALVES", , 30 September 2015 (2015-09-30), XP055323272, Retrieved from the Internet: URL:http://www.coster.com/en_GB/Download/C atalogue/Actuators-Section.pdf [retrieved on 2016-11-25]
- Anonymous: "Aerosol Valves", , 30 September 2002 (2002-09-30), XP055323276, Retrieved from the Internet: URL:http://www.coster.com/en_GB/Download/C atalogue/Valves-Section.pdf [retrieved on 2016-11-25]
- Verville: "Introduction to Aerosol Valve Technology", , 14 March 2007 (2007-03-14), XP55420614, Retrieved from the Internet: URL:http://www.southernaerosol.com/Power Point/Powerpoint pdfs/SATA_Valve_Technology.pdf [retrieved on 2017-10-31]

## Description

### Field of invention

The present invention relates to a "compressed" aerosol hairspray product provided in a can having reduced amount of hair styling composition yet providing the same number of uses as a non-compressed product.

### Background and prior art

Hairstyling products such as hairsprays are used for achieving different hairstyles and for holding hair strands in place for a period of time. Typically, hairsprays comprise film-forming polymers, which when applied to keratin-containing fibres, such as human hair, form fibre-fibre welds. These welds 'glue' the fibres together and hence impart hold to the hairstyle.

Aerosol hairspray products usually comprise a pressure- resistant container, a nozzle, a propellant, and a hairstyling formulation. A hairspray composition is normally ejected from such products via aerosol-forming nozzle. See, for example, US2009/0104138A1.

Environmental sustainability has become a matter of growing interest in aerosol applications, and concentrates have the potential to benefit both manufacturers and the environment. Aerosol concentrates offer manufacturers cost advantages in terms of reduced packaging (both primary and secondary), reduced propellant use, reduced shipping costs, and the like. The environment is benefited by a product that, compared to conventional aerosols, emits less propellant (e.g., volatile organic compound (VOC)), generates less packaging waste and, from manufacture through disposal, has a reduced carbon footprint.

It is, therefore, desirable to reduce the size and volume of pressurised products, such as hairsprays. Such products reduce the amount of hair spray composition per consumer use without impacting performance, thus maintaining the same number of consumer uses per can.

GB 2,299,507 A (Unilever) discloses low flow rate propellant driven antiperspirant compositions comprising less than 60% by weight of a propellant and an initial spray rate of no more than 0.5 g/s.

EP 674,899 B1 (Unilever) discloses concentrated deodorant compositions comprising propellant, most preferably at 30-60% by weight of the composition, and having a discharge valve adapted to allow the composition to be sprayed at an initial spray rate of less than 0.3 g/s.

US2015/0004200 (The Procter & Gamble Company) discloses an aerosol hairspray product with a pressurisable container containing an aqueous, substantially alcohol free hairstyling formulation. A spraying device is attached to the container for dispensing the hairstyling formulation and comprises a valve and a nozzle, wherein the valve comprises a valve body, a stem and a spring means. The valve body houses an insert having an insert orifice and at least two channels, which are tangentially disposed about the insert orifice. The valve body also comprises at least two vapour taps, and the insert orifice is capable of being in liquid communication with the hairstyling formulation in the reservoir. In this context the document WO9110420 is also relevant.

Consumer habits and perceptions can be exceedingly difficult to change. If a product does not fit conventional spray habits, consumer acceptance of that product may be difficult, no matter how good the product. Conversely, a product that matches consumer behaviour but does not deliver on efficacy and sensory requirements is unlikely to be commercially accepted. When providing aerosol products in concentrated form, reference need be made to the manner in which the aerosol is conventionally used. A typical spray period for a hair spray product, by which is meant the time during which an actuator is actively engaged to dispense product, is typically on the order of five seconds.

Producing an acceptable aerosol concentrate requires more than simply changing the level of active, since irrespective of active level, consumers are likely to stick to their conventional application habits. Minimizing spray rate is another approach to concentrates. Typical spray rates for hair spray aerosols are of the order of 0.5 g/s to 1.5 g/s, but lower rates may be desirable for concentrated aerosols. Minimizing spray rate theoretically requires little or no disruption to consumer behavior, because the same dose can then be applied in approximately the same time, despite the aerosol being more concentrated. However, reducing spray rate presents numerous issues for product engineers. As a practical matter, reducing spray rate can exacerbate nozzle clogging and increase spluttering. Additionally, reduced spray rates present challenges in terms of providing compositions that provide acceptable sensory properties, given the confines of consumer application habits and typical application spray periods. Reduced spray rate can also result in reduced product efficacy if insufficient active is dispensed. Therefore, compression not only requires reduced spray rate but also changes to the formulation to ensure that the compressed product is acceptable to the consumer.

One method of achieving compression of spray products is to reduce the spray rate. If the spray rate is reduced, it follows that the concentration of the resin in the formulation should be doubled to compensate. However, doubling the resin in the formulation can lead to significant challenges in terms of stability because there is too much solid content. In addition it can lead to clogging issues in the valve, insert and actuator orifice. These all negatively impact product performance and integrity.

There remains a need for concentrated aerosol hair spray products that provide a combination of desirable sensory properties and dispensing properties, without compromising product efficacy. Such products reduce the amount of hair spray composition per consumer use without impacting performance, thus maintaining the same number of consumer uses per can.

We have now found that a specially developed valve can control the spray rate to achieve compression, in combination with an optimised insert, to achieve the correct spray characteristics of a hair styling formulation. Surprisingly, there is no need to double the resin concentration.

### Definition of the invention

In a first aspect of the invention there is provided an aerosol hairspray product, which comprises:
a) a pressurisable container comprising:
   i) a container wall which encloses a reservoir for storing a hairstyling formulation, and
   ii) a propellant;
b) a hairstyling formulation comprising:
   (iii) from 20 wt % to 75 wt % alcohol, by total weight of the hairstyling formulation and propellant; and
   (iv) from 1.0 wt % to 15 wt % hairstyling polymer, by total weight of the hairstyling formulation and propellant; and
c) a spraying device attached to the container for dispensing the hairstyling formulation from the reservoir of the container, which comprises a valve and an actuator,
wherein the valve comprises a valve body, said valve body comprising a stem and a spring means; and wherein the valve comprises a restricted tail piece (RTP) of diameter 0.1 mm to 2.5 mm, and a vapour phase tap (VPT) of diameter 0.1 mm to 1.5 mm;
wherein the actuator comprises a main spray channel and communicates with an insert, said insert comprising an exit orifice and from 1 to 8 sub-spray channels; wherein said channels are tangentially disposed about the exit orifice;
wherein the exit orifice is capable of being in liquid communication with the hairstyling formulation in the reservoir;
characterised in that the exit orifice has a diameter of from 0.01 to 2 mm; and an orifice thickness of from 0.1 to 1.5 mm;
and wherein the sub-spray channels have a height of from 0.1 to 0.8 mm and a length of from 0.1 to 0.5mm.

In a second aspect of the invention there is provided a method of treating hair with a hairspray product of the aspect of the invention, comprising the step of applying to the hair a hair styling formulation as defined in the first aspect of the invention, using a hair spray product of the first aspect of the invention.

### Brief Description of the Figures

Embodiments of the invention will now be described with reference to the following non-limiting drawings in which:
Figure 1 is a perspective view of a hairspray product in accordance with the invention.
Figure 2 is a cross section view of a spraying device (4) used in the hairspray product of the invention.
Figure 3 is a cross section view of a valve (5) used in the hairspray product of the invention, where the valve is installed in the container (seated in a valve cup (16)).
Figure 4 (a) is a cross section view of an insert (6) as used in the hairspray product of the invention.
Figure 4 (b) is a cross sectional view of an insert (6) for use in the hairspray of the invention.

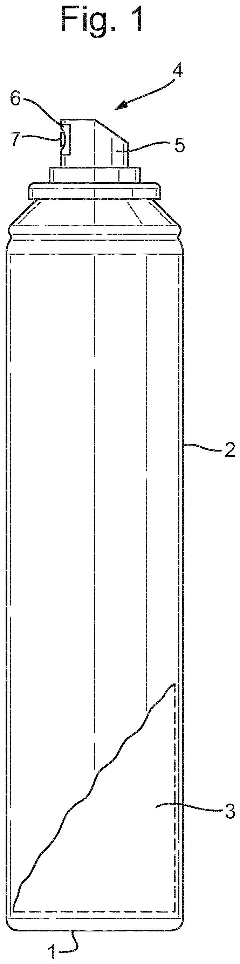

Figure 1 is a perspective view of a hairspray product comprising a pressurisable container (1) having a container wall (2) and a reservoir (3); with a spraying device (4) comprising an actuator (5) having an insert (6) with an exit orifice (7).

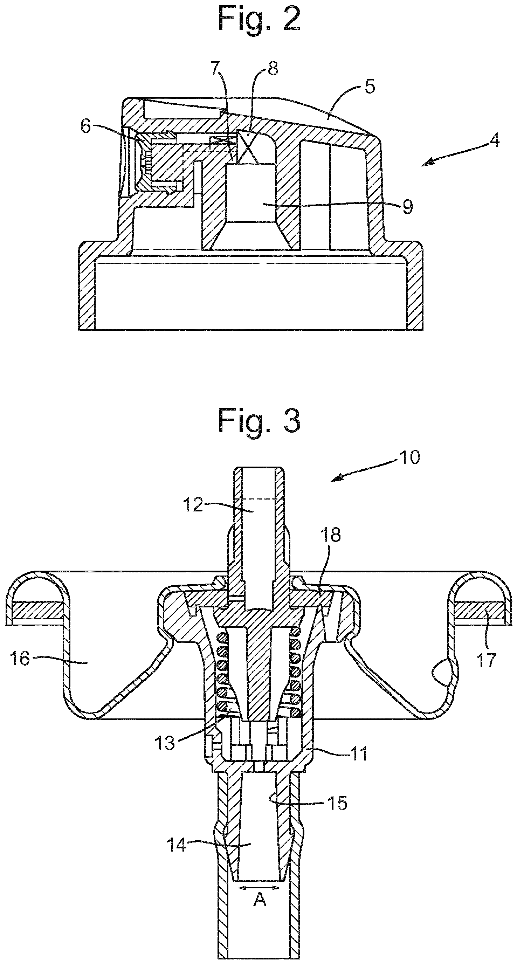

Figure 2 is a cross section view of a spraying device (4) comprising an actuator (5) having a main spray channel (8) and an insert (6) with an exit orifice (7). A stem socket is also shown.

Figure 3 is a cross sectional view of a valve (10) used in the hairspray product of the invention, where the valve is installed in the container, seated in a valve cup (16). The valve (10) comprises a valve body (11), said valve body comprising a stem (12) and a spring means (13); and wherein the valve has a restricted tail piece (RTP) (14) with a diameter indicated by a double ended arrow "A", and a vapour phase tap (VPT) (15). The valve cup (16) comprises an outer gasket (17) and an inner gasket (18).

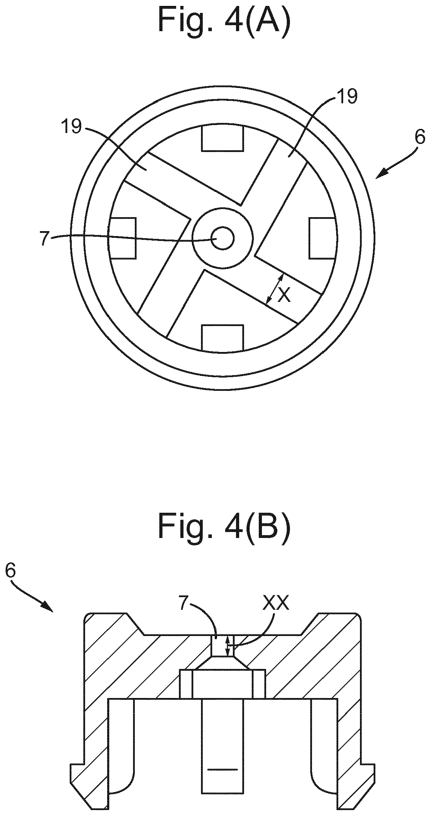

Figure 4a is a cross section view of an insert (6) comprising an exit orifice (7) and four sub-spray channels (19); wherein said channels are tangentially disposed about the exit orifice (7). Double ended arrow "X" indicates the length of the sub-spray channel as defined in claim 1.

Figure 4b is a cross section view of an insert (6) showing the exit orifice (7). Double ended arrow "XX" indicates the orifice thickness as defined in claim 1.

### General description of the Invention

### The aerosol hairspray product

The product relates to an aerosol hairspray product comprising: a container comprising a reservoir for a hairstyling formulation and a liquefied gas propellant, and a spraying device attached to the container.

In at least one embodiment, the product comprises from 50% to 65% hairstyling formulation, or from 55% to 60% hairstyling formulation, by total weight of the hairstyling formulation and propellant.

In at least one embodiment, the product comprises from 35% to 50% propellant, or from 40% to 45% propellant, by total weight of the hairstyling formulation and propellant.

As used herein, unless otherwise stated, details of the hairstyling formulation refers to the formulation before it is placed into the container whereupon it will mix to an extent with the propellant since the propellant is a liquefied gas propellant. The same is true where details of the propellant are mentioned, unless otherwise stated.

The product is an aerosol hairspray product and thus does not include mousse products or any pump spray products.

Conventional spray rates are typically in the range of from 0.5 to 1.5 g/sec. The product of the invention delivers 80 to 20 % of this range, preferably 70 to 30 %, most preferably from 60 to 40 % of this spray rate. This reduction in spray rate without loss of performance is one of the advantages of this invention. Therefore, the aerosol hairspray product sprays at a delivery rate of from 80 to 20 %, preferably from 70 to 30 %, most preferably from 60 to 40 % of 0.5 to 1.5 g/sec.

### The Pressurisable Container

The pressurisable container comprises a container wall which encloses a reservoir for storing a hairstyling formulation, and a propellant.

The container wall may be predominantly straight, curved or tapered.

The container can be any size, but is preferably a "compressed" size or travel size.

The container preferably has a volume (so-called overfill capacity) of from 200 to 300 ml, more preferably from 220 to 280 ml, most preferably from 230 to 260 ml.

The container preferably has a diameter of from 35 to 50 mm, more preferably from 40 to 45 mm.

The container preferably has a height of from 80 to 220 mm, preferably from 90 to 180 mm, most preferably from 110 to 150 mm.

### The propellant

The hairspray composition further comprises a propellant. Preferred propellants are selected from hydrofluorocarbons (for example hydrofluorocarbon 152A), dimethylether, hydrocarbons such as propane, isobutane, butane and mixtures thereof, and compressed gasses such as nitrogen and carbon dioxide and mixtures thereof.

### The hairstyling formulation

The product comprises a hairstyling formulation comprising a hair styling polymer and a neutraliser in an alcoholic composition. It is well known in the art that a water based composition leads to consumer negative performance such as curl droop, long drying times and stickiness on the hair. These negatives are not experienced with alcoholic compositions.

### The hair styling polymer

The hairstyling polymer is present in an amount of from 1.0 to 10 wt %, preferably from 1.0 to 8.0 wt %, most preferably from 2 to 7 wt % by total weight of the hairstyling formulation and propellant.

The hairstyling polymer may be selected from a VA/Crotonates/Vinyl Neodecanoate Copolymer, Polyurethane-14 (and) AMP-Acrylates Copolymer (for example, DynamX available from AzkoNoble), Butyl Ester of PVM/MA Copolymer (for example, Gantrez available from Ashland), Polyester-5, Octylacrylamide/Acrylates/Butylaminoethyl Methacrylate Copolymer, imidized isobutylene/maleic anhydride copolymer (for example, Aquaflex™ FX-64 available from Ashland), Polyvinylpyrrolidone, Acrylates/Hydroxyesters Acrylates Copolymer (for example, Acudyne 180 available from DOW), Polyurethane-6, Acrylates/Octylacrylamide Copolymer and Acrylates Copolymer (for example, Balance CR available from AkzoNoble and Luvimer 100 P available from BASF), and more preferably from VA/Crotonates/Vinyl Neodecanoate Copolymer (for example, Resyn 28-2930 available from AkzoNoble) and Octylacrylamide/Acrylates/Butylaminoethyl Methacrylate Copolymer (for example, Amphomer available from AkzoNoble).

Preferably the styling resin comprises VA/Crotonates/Vinyl Neodecanoate Copolymer (for example, Resyn 28-2930 available from AkzoNoble) and Octylacrylamide/Acrylates/Butylaminoethyl Methacrylate Copolymer (for example, Amphomer available from AkzoNoble).

### The neutralizer

The neutralizer provides an alkaline base. The neutralizer is preferably selected from potassium hydroxide, sodium hydroxide, triisopropanolamine, 2-aminobutanol, 2-aminomethyl propanol, aminoethylpropandiol, dimethyl stearamine, sodium silicate, tetrahydroxypropyl ethylenediamine, ammonia, triethanolamine, trimethylamine, aminomerhylpropandiol, and more preferably 2-aminomethyl propanol, dimethyl stearamine and 2-aminobutanol and mixtures thereof.

### The alcohol

The alcohol is preferably selected from ethanol, isopropyl alcohol, butanol, most preferably ethanol.

The alcohol is present in an amount of from 30 to 75 wt % by total weight of the hairstyling formulation and propellant, preferably from 35 to 70 wt %, more preferably from 35 to 60 wt %, most preferably from 35 to 55 wt %, by total weight of the hairstyling formulation and propellant.

### Substantially water free

The hairstyling formulation is preferably substantially free of water. The hair styling formulation preferably comprises less than 2 wt % water, by total weight of the hairstyling formulation and propellant. In at least one embodiment, the hairstyling formulation comprises 0.001 to 1.8 wt %, or 0.001 to 1.5 wt %, or 0.001 to 1 wt % water by total weight of the hairstyling formulation and propellant. Most preferably, the formulation is anhydrous.

### Additional ingredients

The formulation may also comprise other adjuncts commonly used in hair sprays. Preferably perfume, silicone (for example phenyl trimethicone, cyclopentylsiloxane, PEG/PPG-20/15 Dimethicone), emotives, corrosion inhibitors, emollients, UV absorbers (for example benzopheonone-4, ethyl hexyl methoxy cinnamate) and mixtures thereof.

### The Spraying Device

Attached to the container is a spraying device for spraying the hairstyling formulation onto a substrate.

### Valve

The spraying device comprises a valve.

The valve comprises a valve body, said valve body comprising a stem and a spring means.

The valve comprises a restricted tail piece (RTP) of diameter 0.1 mm to 2.5 mm, preferably 0.2 to 1.5 mm, most preferably 0.3 to 1.0 mm.

The valve comprises a vapour phase tap (VPT) of diameter 0.1 mm to 1.5 mm, preferably 0.2 to 1.0 mm, most preferably 0.3 to 0.8 mm.

When installed in the container, the valve is preferably seated in a valve cup (16). The valve cup (16) preferably comprises an outer gasket (17), which enables connection to the can by crimping onto a can bead; and an inner gasket (18). The inner gasket controls the release product when the actuator is pressed.

### Actuator

The spraying device comprises an actuator. The actuator typically sits over the stem.

The actuator comprises a main spray channel and communicates with an insert.

### Insert

The insert comprises an exit orifice and from 1 to 8 sub-spray channels, preferably 4 to 6 sub-spray channels, most preferably 6 sub-spray channels; wherein said channels are tangentially disposed about the exit orifice.

Suitable inserts are available from, for example, Coster.

The insert controls the cone angle. A wide cone angle is preferred whilst maintaining the desirable particle size distribution.

### Channels

The sub-spray channels have a height of from 0.1 to 0.8, preferably from 0.3 to 0.7 mm and a length of from 0.1 to 0.5, preferably from 0.25 to 0.35 mm.

### Exit Orifice

The exit orifice is capable of being in liquid communication with the hairstyling formulation in the reservoir.

The exit orifice has a diameter of from 0.01 to 2 mm, most preferably 0.5 mm; and an orifice thickness of from 0.1 to 1.5 mm, preferably from 0.3 to 1.0 mm;

The orifice may be straight or conical in shape.

### Examples

The following hair sprays were prepared:
Hair Spray 1, in accordance with the invention.
Hair Spray A, which was a comparative hair spray.
Details of the hairstyling formulations and the spraying device features are given in Table 1 below.

The performance of Hair Sprays 1 and A was assessed using a standard half head mannequin test protocol. The mannequin head (with hair no more than 12 cm in length) was first prepared using a lightweight shampoo and conditioner and blow-dried into a straight smooth style. The hair sprays were shaken gently before each use.

The comparative Hair Spray A was sprayed for 10 seconds evenly over the hair on one side of the mannequin head from a distance of 30 cm. This was repeated on the other side of the mannequin head using Hair Spray 1 in accordance with the invention. A perspective divider was placed down the centre of the mannequin to ensure there was no cross contamination during the hair spray application.

The performance attributes of Hold, Flexibility and Softness were evaluated by expert graders using a 1 to 9 point scale with half units for each attribute (wherein the higher rating would be more desirable).

Hold was defined as how well the hair holds the style post spraying with a tactile assessment by expert graders.

Softness was assessed by the expert graders using their hands to scrunch the hair in their palms whilst evaluating the feel of softness.

Flexibility was defined as the ability for the hair to move and bend without disruption of the style and was assessed by gently swinging the head from side to side 3 times, and the expert grader visually assessing both movement and hold.

Results are given in Table 1 below.

**Table 1:**

| | **Comparative Hair Spray A** | **Inventive Hair Spray 1** |
|---|---|---|
| **Spraying Device** | | |
| Valve RTP | 0.33 mm | 0.8mm |
| Valve VPT | None | 0.5mm |
| Valve STEM | 0.33 mm | 0.3mm |
| Actuator (type & orifice) | Aptar 0.64 mm | Coster 0.5 mm |
| Actuator No. of channels | n/a | 6 |
| | | |
| **Spray rate** | 100% | 50% |
| | | |

| **Hairstyling Formulation** | | |
|---|---|---|
| Total hairstyling Polymer | 4 - 5% | 5.5% |
| Ethanol | 53% | 53% |
| Propellant | 40% | 40% |
| Minors (fragrance, pH modifiers) | 2 - 3% | 1.5% |

| **Performance** | | |
|---|---|---|
| Hold | 7.0 | 6.5 |
| Flexibility | 5.5 | 5.0 |
| Softness | 3.0 | 3.0 |

The results clearly show that the inventive example 1 achieves the same performance with a 50 % reduced spray rates compared to the comparative example A.

## Claims

1. An aerosol hairspray product, which comprises:
a) a pressurisable container comprising
i) a container wall which encloses a reservoir for storing a hairstyling formulation, and
ii) a propellant;
b) a hairstyling formulation comprising:
(iii) from 20 wt % to 75 wt % alcohol, by total weight of the hairstyling formulation and propellant; and
(iv) from 1.0 wt % to 15 wt % hairstyling polymer, by total weight of the hairstyling formulation and propellant; and
c) a spraying device attached to the container for dispensing the hairstyling formulation from the reservoir of the container, which comprises a valve and an actuator,
wherein the valve comprises a valve body, said valve body comprising a stem and a spring means; and wherein the valve comprises a restricted tail piece (RTP) of diameter 0.1 mm to 2.5 mm, and a vapour phase tap (VPT) of diameter 0.1 mm to 1.5 mm;
wherein the actuator comprises a main spray channel and communicates with an insert, said insert comprising an exit orifice and from 1 to 8 sub-spray channels; wherein said channels are tangentially disposed about the exit orifice;
wherein the exit orifice is capable of being in liquid communication with the hairstyling formulation in the reservoir;
**characterised in that** the exit orifice has a diameter of from 0.01 to 2 mm; and an orifice thickness of from 0.1 to 1.5 mm;
and wherein the sub-spray channels have a height of from 0.1 to 0.8 mm and a length of from 0.1 to 0.5 mm.

2. An aerosol hairspray product as claimed in claim 1, comprises less than 2 wt % water, by total weight of the hairstyling formulation and propellant.

3. An aerosol hairspray product as claimed in claim 1 or claim 2, wherein the formulation is anhydrous.

4. An aerosol hairspray product as claimed in any preceding claim, wherein the hairstyling polymer is present in an amount of from 1.0 to 10 wt by total weight of the hairstyling formulation and propellant.

5. An aerosol hairspray product as claimed in any preceding claim, wherein the styling polymer is selected from a VA/Crotonates/Vinyl Neodecanoate Copolymer, Polyurethane-14 (and) AMP-Acrylates Copolymer, Butyl Ester of PVM/MA Copolymer, Polyester-5, Octylacrylamide/Acrylates/Butylaminoethyl Methacrylate Copolymer, Acrylates/Hydroxyesters Acrylates Copolymer, Polyurethane-6, Acrylates/Octylacrylamide Copolymer and Acrylates Copolymer, and more preferably from VA/Crotonates/Vinyl Neodecanoate Copolymer and Octylacrylamide/Acrylates/Butylaminoethyl Methacrylate Copolymer.

6. An aerosol hairspray product as claimed in any preceding claim, wherein the formulation comprises an alkaline base.

7. An aerosol hairspray product as claimed in any preceding claim, wherein the RTP has a diameter of from 0.2 to 1.5 mm, preferably from 0.3 to 1.0 mm.

8. An aerosol hairspray product as claimed in any preceding claim, wherein the VPT has a diameter of from is from 0.2 to 1.0 mm, preferably from 0.3 to 0.8 mm.

9. An aerosol hairspray product as claimed in any preceding claim, wherein the container has a volume of from 200 to 300 ml.

10. An aerosol hairspray product as claimed in any preceding claim, wherein the exit orifice comprises 6 sub-spray channels.

11. An aerosol hairspray product as claimed in any preceding claim, wherein the sub-spray channels have a height of from 0.3 to 0.7 mm.

12. An aerosol hairspray product as claimed in any preceding claim, wherein the sub-spray channels have a length of from 0.25 to 0.35 mm.

13. An aerosol hairspray product as claimed in any preceding claim, wherein the exit orifice has an orifice thickness of from 0.1 to 1.5 mm.

14. A method of treating hair with a hairspray product as defined in any one of claims 1 to 13, comprising the step of applying to the hair a hair styling formulation as defined in any one of claims 1 to 6, using a hair spray product as defined in any of claims 1 to 13.

## Patentansprüche

1. Haarspray-Aerosolprodukt, das Folgendes umfasst:
a) einen Behälter, der mit Druck beaufschlagt werden kann, der Folgendes umfasst:
i) eine Behälterwand, die einen Sammelbehälter zum Aufbewahren einer Haarstyling-Rezeptur umfasst, und
ii) ein Treibmittel;
b) eine Haarstyling-Rezeptur, die Folgendes umfasst:
(iii) von 20 Gew.-% bis 75 Gew.-% Alkohol in Bezug auf das Gesamtgewicht der Haarstyling-Rezeptur und des Treibmittels; und
(iv) von 1,0 Gew.-% bis 15 Gew.-% Haarstyling-Polymer in Bezug auf das Gesamtgewicht der Haarstyling-Rezeptur und des Treibmittels; und
c) eine Sprühvorrichtung, die an dem Behälter befestigt ist, zum Ausgeben der Haarstyling-Rezeptur aus dem Sammelbehälter des Behälters, die ein Ventil und einen Aktuator umfasst,
wobei das Ventil einen Ventilkörper umfasst, wobei der Ventilkörper einen Stiel und Federmittel umfasst; und wobei das Ventil ein begrenztes Schlussstück (RTP) mit einem Durchmesser im Bereich von 0,1 mm bis 2,5 mm und einen Dampfphasen-Abgriff (VPT) mit einem Durchmesser im Bereich von 0,1 mm bis 1,5 mm umfasst;
wobei der Aktuator einen Hauptsprühkanal umfasst und mit einem Einsatz kommuniziert, wobei der Einsatz eine Austrittsöffnung und 1 bis 8 Nebensprühkanäle umfasst; wobei die Kanäle um die Austrittsöffnung tangential angeordnet sind;
wobei die Austrittsöffnung in Flüssigkeitskommunikation mit der Haarstyling-Rezeptur in dem Sammelbehälter sein kann;
**dadurch gekennzeichnet, dass** die Austrittsöffnung einen Durchmesser im Bereich von 0,01 bis 2 mm; und eine Öffnungsdicke im Bereich von 0,1 bis 1,5 mm aufweist;
und wobei die Nebensprühkanäle eine Höhe im Bereich von 0,1 bis 0,8 mm und eine Länge im Bereich von 0,1 bis 0,5 mm aufweisen.

2. Haarspray-Aerosolprodukt nach Anspruch 1, das weniger als 2 Gew.-% Wasser in Bezug auf das Gesamtgewicht der Haarstyling-Rezeptur und des Treibmittels umfasst.

3. Haarspray-Aerosolprodukt nach Anspruch 1 oder Anspruch 2, wobei die Rezeptur wasserfrei ist.

4. Haarspray-Aerosolprodukt nach einem der vorhergehenden Ansprüche, wobei das Haarstyling-Polymer in einer Menge im Bereich von 1,0 bis 10 Gew.-% in Bezug auf das Gesamtgewicht der Haarstyling-Rezeptur und des Treibmittels vorliegt.

5. Haarspray-Aerosolprodukt nach einem der vorhergehenden Ansprüche, wobei das Styling-Polymer aus VA/Crotonat/Vinylneodecanoat-Copolymer, Polyurethan-14 (und) AMP-Acrylat-Copolymer, Butylester von PVM/MA-Copolymer, Polyester-5, Octylacrylamid/Acrylat/Butylaminoethylmethacrylat-Copolymer, Acrylat/Hydroxyester Acrylat-Copolymer, Polyurethan-6, Acrylat/Octylacrylamid-Copolymer und Acrylat-Copolymer, und stärker bevorzugt aus VA/Crotonat/Vinylneodecanoat-Copolymer und Octylacrylamid/Acrylat/Butylaminoethylmethacrylat-Copolymer ausgewählt wird.

6. Haarspray-Aerosolprodukt nach einem der vorhergehenden Ansprüche, wobei die Rezeptur eine basische Basis umfasst.

7. Haarspray-Aerosolprodukt nach einem der vorhergehenden Ansprüche, wobei das RTP einen Durchmesser im Bereich von 0,2 bis 1,5 mm, vorzugsweise im Bereich von 0,3 bis 1,0 mm aufweist.

8. Haarspray-Aerosolprodukt nach einem der vorhergehenden Ansprüche, wobei das VPT einen Durchmesser im Bereich von 0,2 bis 1,0 mm, vorzugsweise im Bereich von 0,3 bis 0,8 mm aufweist.

9. Haarspray-Aerosolprodukt nach einem der vorhergehenden Ansprüche, wobei der Behälter ein Volumen im Bereich von 200 bis 300 ml aufweist.

10. Haarspray-Aerosolprodukt nach einem der vorhergehenden Ansprüche, wobei die Austrittsöffnung 6 Nebensprühkanäle umfasst.

11. Haarspray-Aerosolprodukt nach einem der vorhergehenden Ansprüche, wobei die Nebensprühkanäle eine Höhe im Bereich von 0,3 bis 0,7 mm aufweisen.

12. Haarspray-Aerosolprodukt nach einem der vorhergehenden Ansprüche, wobei die Nebensprühkanäle eine Länge im Bereich von 0,25 bis 0,35 mm aufweisen.

13. Haarspray-Aerosolprodukt nach einem der vorhergehenden Ansprüche, wobei die Austrittsöffnung eine Öffnungsdicke im Bereich von 0,1 bis 1,5 mm aufweist.

14. Verfahren zum Behandeln von Haar mit einem Haarspray-Produkt nach einem der Ansprüche 1 bis 13, das den Schritt des Aufbringens einer Haarstyling-Rezeptur nach einem der Ansprüche 1 bis 6 auf das Haar unter Verwendung eines Haarspray-Produkts nach einem der Ansprüche 1 bis 13 umfasst.

## Revendications

1. Produit de laque en aérosol, qui comprend :
a) un récipient pressurisable comprenant
i) une paroi de récipient qui enferme un réservoir pour stocker une formulation de coiffage, et
ii) un gaz propulseur ;
b) une formulation de coiffage comprenant :
(iii) de 20 % en masse à 75 % en masse d'alcool, en masse totale de la formulation de coiffage et de gaz propulseur ; et
(iv) de 1,0 % en masse à 15 % en masse de polymère de coiffage, en masse totale de la formulation de coiffage et de gaz propulseur ; et
c) un dispositif de pulvérisation fixé au récipient pour distribuer la formulation de coiffage à partir du réservoir du récipient, qui comprend une vanne et un actionneur,
dans lequel la vanne comprend un corps de vanne, ledit corps de vanne comprenant une tige et un moyen de ressort ; et dans lequel la vanne comprend une pièce de queue restreinte (RTP) de diamètre 0,1 mm à 2,5 mm, et un robinet de phase vapeur (VPT) de diamètre 0,1 mm à 1,5 mm ;
dans lequel l'actionneur comprend un canal de pulvérisation principal et communique avec un insert, ledit insert comprenant un orifice de sortie et de 1 à 8 sous-canaux de pulvérisation ; dans lequel lesdits canaux sont tangentiellement disposés autour de l'orifice de sortie ;
dans lequel l'orifice de sortie peut être en communication liquide avec la formulation de coiffage dans le réservoir ;
**caractérisé en ce que** l'orifice de sortie présente un diamètre de 0,01 à 2 mm ; et une épaisseur d'orifice de 0,1 à 1,5 mm ;
et dans lequel les sous-canaux de pulvérisation présentent une hauteur de 0,1 à 0,8 mm et une longueur de 0,1 à 0,5 mm.

2. Produit de laque en aérosol selon la revendication 1, qui comprend moins de 2 % en masse d'eau, en masse totale de la formulation de coiffage et de gaz propulseur.

3. Produit de coiffage en aérosol selon la revendication 1 ou la revendication 2, dans lequel la formulation est anhydre.

4. Produit de coiffage en aérosol selon l'une quelconque des revendications précédentes, dans lequel le polymère de coiffage est présent dans une quantité de 1,0 à 10 % en masse en masse totale de la formulation de coiffage et de gaz propulseur.

5. Produit de laque en aérosol selon l'une quelconque des revendications précédentes, dans lequel le polymère de coiffage est choisi parmi un copolymère de VA/crotonates/néodécanoate de vinyle, copolymère de polyuréthane-14 (et) AMP-acrylates, ester butylique de copolymère de PVM/MA, polyester-5, copolymère d'octylacrylamide/ acrylates/méthacrylate de butylaminoéthyle, copolymère d'acrylates/ acrylates d'hydroxyesters, polyuréthane-6, copolymère d'acrylates/ octylacrylamide et copolymère d'acrylates, et encore mieux parmi un copolymère de VA/crotonates/néodécanoate de vinyle et copolymère d'octylacrylamide/acrylates/méthacrylate de butylaminoéthyle.

6. Produit de laque en aérosol selon l'une quelconque des revendications précédentes, dans lequel la formulation comprend une base alcaline.

7. Produit de laque en aérosol selon l'une quelconque des revendications précédentes, dans lequel le RTP présente un diamètre de 0,2 à 1,5 mm, de préférence de 0,3 à 1,0 mm.

8. Produit de laque en aérosol selon l'une quelconque des revendications précédentes, dans lequel le VPT présente un diamètre de 0,2 à 1,0 mm, de préférence de 0,3 à 0,8 mm.

9. Produit de laque en aérosol selon l'une quelconque des revendications précédentes, dans lequel le récipient présente un volume de 200 à 300 ml.

10. Produit de laque en aérosol selon l'une quelconque des revendications précédentes, dans lequel l'orifice de sortie comprend 6 sous-canaux de pulvérisation.

11. Produit de laque en aérosol selon l'une quelconque des revendications précédentes, dans lequel les sous-canaux de pulvérisation présentent une hauteur de 0,3 à 0,7 mm.

12. Produit de laque en aérosol selon l'une quelconque des revendications précédentes, dans lequel les sous-canaux de pulvérisation présentent une longueur de 0,25 à 0,35 mm.

13. Produit de laque en aérosol selon l'une quelconque des revendications précédentes, dans lequel l'orifice de sortie présente une épaisseur d'orifice de 0,1 à 1,5 mm.

14. Procédé de traitement des cheveux avec un produit de laque selon l'une quelconque des revendications 1 à 13, comprenant l'étape d'application aux cheveux d'une formulation de coiffage des cheveux comme définie dans l'une quelconque des revendications 1 à 6, en utilisant un produit de laque comme défini dans l'une quelconque des revendications 1 à 13.
